# EUROPEAN PATENT SPECIFICATION

(11) **EP 1 713 801 B1**
(45) Date of publication and mention of the grant of the patent: **12.12.2007**
(21) Application number: 05702373.1
(22) Date of filing: 26.01.2005
(51) Int. Cl.: C07D 453/02

(54) **PROCESS FOR PREPARATION OF 1-(2S,3S)-2-BENZHYDR YL-N-(5-TERT-BUTYL-2-METHOXYBENZYL)QUINUCLIDIN-3-AMINE**
VERFAHREN ZUR HERSTELLUNG VON 1-(2S,3S)-2-BENZHYDRIL-N-(5-TERT.-BUTYL-2-METHOXYBENZYL)CHINUKLIDIN-3-AMIN
PROCEDE DE PREPARATION DE 1-(2S,3S)-2-BENZHYDRYL-N-(5-TERT-BUTYL-2-METHOXYBENZYL)QUINUCLIDINE-3-AMINE

(30) Priority: 02.02.2004 US 541323 P
(43) Date of publication of application: 25.10.2006
(73) Proprietor: Pfizer Products Incorporated, Groton, CT 06340 (US)
(72) Inventor: BASFORD, Patricia Ann, Pfizer Global R&D, Sandwich, Kent CT13 9NJ (GB); POST, Ronald James, Pfizer Global R&D, Groton, CT 06340 (US); SMITH, Julian Duncan, Pfizer Global R&D, Sandwich, Kent CT13 9NJ (GB); TABER, Geraldine Patricia, Pfizer Global R&D, Groton, CT 06340 (US)
(74) Representative: Rutt, Jason Edward
(86) International application number: PCT/IB2005/000221
(87) International publication number: WO 2005/075473

(56) References cited:
- WO-A-97/03984
- WO-A-20/04035575
- US-B1- 6 222 038
- US-B1- 6 255 320
- WARAWA E J ET AL: "Quinuclidine chemistry. 4. Diuretic properties of cis-3-amino-2-benzhydrylquinuclidine." JOURNAL OF MEDICINAL CHEMISTRY. JUN 1975, vol. 18, no. 6, June 1975 (1975-06), pages 587-593, XP002327149 ISSN: 0022-2623

## Description

### FIELD OF INVENTION

This invention relates to an improved process for the preparation of (2S,3S)-2-benzhydryl-*N*-(5-*tert*-butyl-2-methoxybenzyl)quinuclidin-3-amine, (hereinafter "compound of Formula I") and its pharmaceutically acceptable salts. In particular, the invention is directed to an improved synthesis of the monohydrate citrate salt of the compound of Formula **Ia.**

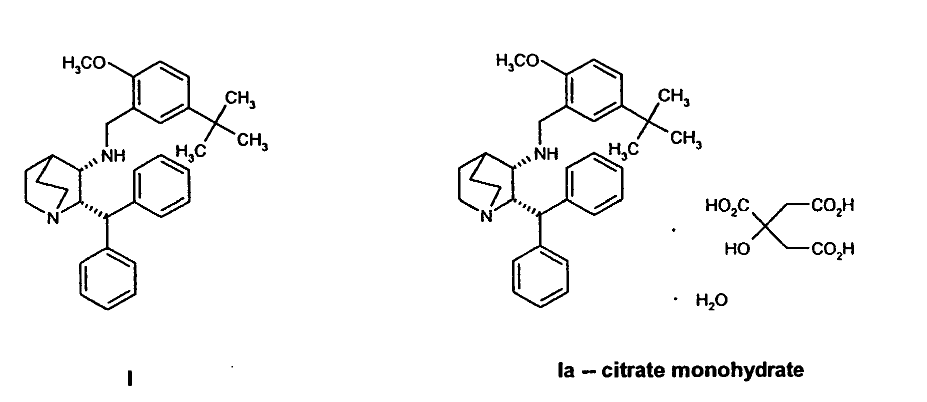

### BACKGROUND OF INVENTION

The compound of Formula **I,** an NK1 receptor antagonist, is effective as an anti-emetic agent for mammals. The compound of Formula I is the subject of U.S. 6,222,038 and U.S. 6,255,320, and the preparation of the compound of Formula I is described therein. U.S. 5,393,762 also describes pharmaceutical compositions and treatment of emesis using NK-1 receptor antagonists. The multiple-use formulation of the compound of Formula I may be parenterally administrated for about five days at the same site for treatment of emesis or other indications. Intravenous or, preferably, subcutaneous administration is desirable for acute use, since retention and absorption of an oral dosage form may be problematic during bouts of emesis. The multiple-use formulation is described in a co-pending U.S. provisional application No. 60/540897 assigned to and owned by Pfizer, Inc.

The compound of Formula I also improves anesthesia recovery in mammals. A co-pending U.S. provisional application No. 60/540,697 assigned to and owned by Pfizer Inc., describes a method of improving anesthesia recovery by administering a NK-1 antagonist prior to, during or after the administration of general anesthesia.

The text of the aforementioned applications and all other references cited in this specification are hereby incorporated by reference in their entirety.

Certain steps within the process description for synthesis of the monohydrate citrate salt of the compound of Formula I were conducted with reagents that are undesirable from a safety perspective and provided unsatisfactory yields for operation on a commercial scale. The present invention is directed to a process whereby the chemical conversions are carried out without the need for aggressive deprotection conditions, aggressive Schiff base-forming conditions or aggressive reducing agents, thus improving the intermediate and product quality and yield. The overall process is improved by use of common solvents for the key chemical conversion steps, by reduction in the number of intermediates requiring isolation, culminating in an overall yield improvement. Further efficiency was achieved by the ability to generate high enantiomeric - purity for the starting product (**VIa**), eliminating purification steps later in the process. Finally, the conditions used in the final step to manufacture the compound of Formula la have been optimized for the generation of the desired morphic form of the mono-citrate monohydrate salt of Compound of Formula I.

### SUMMARY OF INVENTION

In one aspect, the invention is directed to a process for preparing the compound of Formula **Ib,** 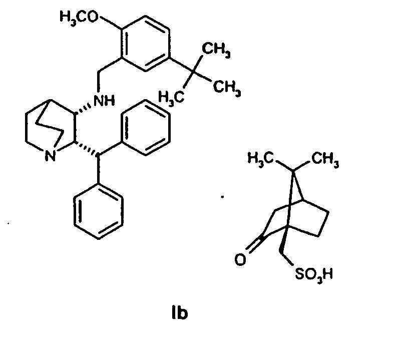 comprising:
(a) deprotecting a compound of Formula **VIa,** 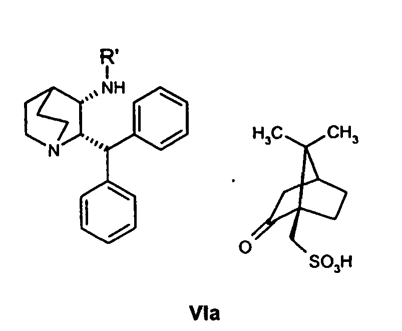 wherein R' is a protecting group, to provide a compound of Formula **VII;** 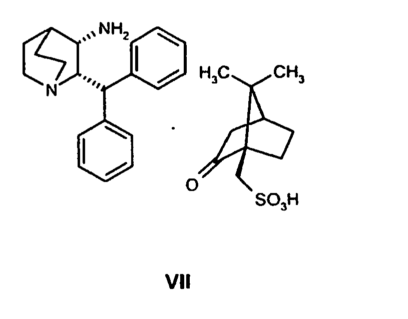
(b) reacting the compound of formula **VII** so formed with a compound of formula 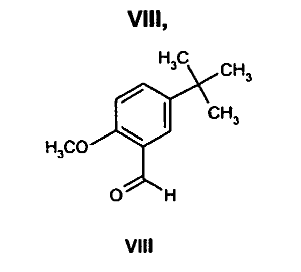 and performing a reductive amination to provide a compound of Formula **Ib,**

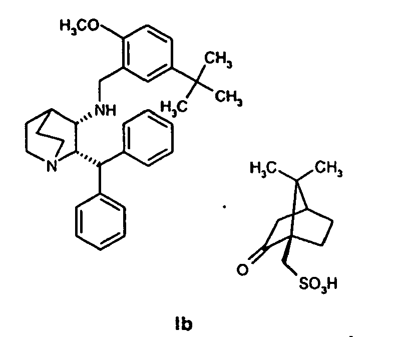

In one embodiment, the invention further comprises removing the camphorsulfonate salt of the compound of Formula **Ib** to provide a compound of Formula **I,**

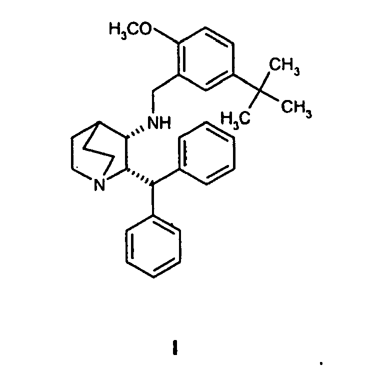

In a preferred embodiment, the protecting group is benzyl, 4-methoxybenzyl, 2,4-dimethoxybenzyl, or triphenylmethyl. Preferably, the deprotection is performed by catalytic hydrogenolysis with hydrogen. Preferably, the catalyst is palladium on carbon, platinum on carbon, palladium on calcium carbonate, or palladium on alumina (Al₂O₃).

In a preferred embodiment, the reductive animation is performed by formation of an imine followed by catalytic hydrogenation. Preferably, the hydrogenation catalyst is palladium on carbon, platinum on carbon, palladium on calcium carbonate, or palladium on alumina (Al₂O₃).

In a preferred embodiment, the process further comprises treating the compound of Formula I with citric acid, forming the compound of Formula **Ia.**

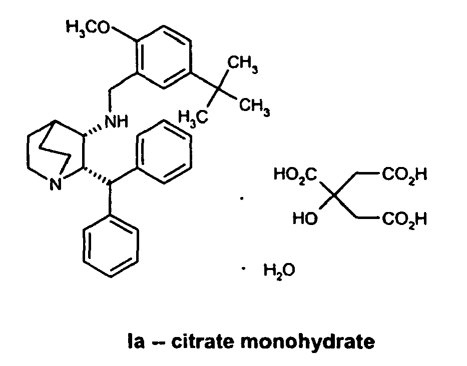

In a second aspect, the invention is directed to a process for preparing the compound of Formula **I,** 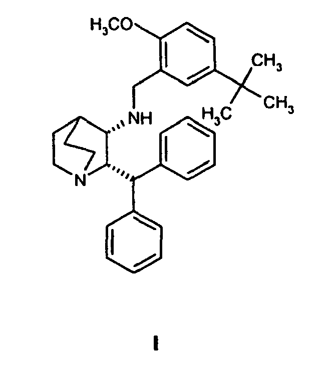 comprising:
(a) debenzylating a compound of Formula **VIa** 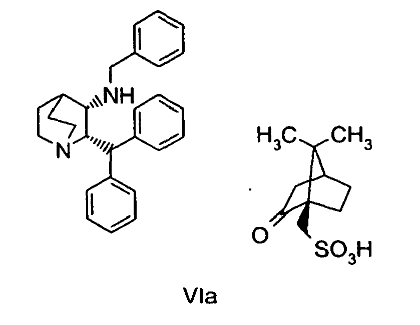 to provide a compound of Formula **VII;** 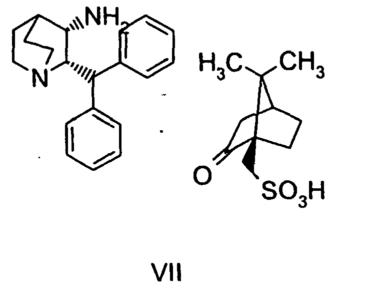
(b) reacting the compound of formula **VII** so formed with a compound of formula **VIII,** 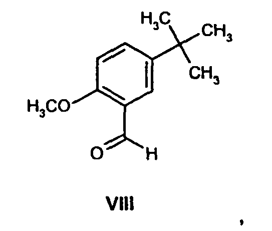 and performing a reductive amination to provide a compound of Formula **Ib,** 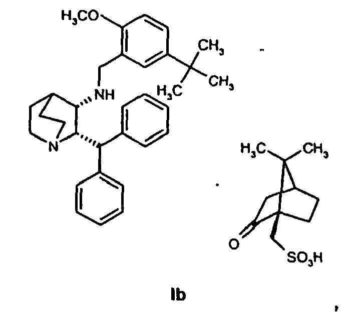 and
(c) removing the camphorsulfonate salt of the compound of **Ib** to provide the compound of Formula I.

In a preferred embodiment, the debenzylation is performed by catalytic hydrogenation. Preferably, the catalyst is palladium on carbon, platinum on carbon, palladium on calcium carbonate, or palladium on alumina (Al₂O₃).

In a preferred embodiment, the process further comprises a reductive amination of step (b) that is performed by catalytic hydrogenation. Preferably, the catalyst is palladium on carbon, platinum on carbon, palladium on calcium carbonate, or palladium on alumina (Al₂O₃).

In another embodiment, the process further comprises isolating the compound of Formula **I.** Preferably, the isolation of the compound of Formula **I** occurs by acid counter ion exchange or basification followed by selective crystallization. Preferably, the crystallization is accomplished in a solvent selected from water, alcohols, ethers, hydrocarbons or mixtures thereof. Preferably, the solvent is isopropanol, toluene or water or mixtures thereof.

In a preferred embodiment, the basification is performed by the addition of an inorganic or organic reagent. Preferably, the reagent is sodium hydroxide, sodium carbonate or sodium bicarbonate.

In another embodiment, the process further comprises treating the compound of Formula **I** with citric acid, forming the compound of Formula **la**

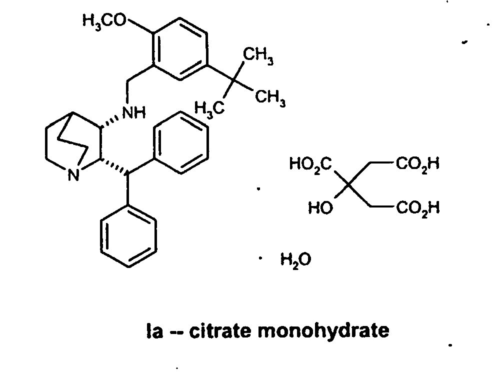

In a preferred embodiment, the process further comprises the addition of acetone and water. Preferably, the process further comprises
a) filtering the solution; and
b) adding a filtered ether solvent,
providing a compound of Formula **Ia.**

In another embodiment, the process further comprising the additional step (c) of granulating the compound of Formula **Ia.** Preferably, the ether solvent is tert-butyl methyl ether. Preferably, the process further comprises applying heat at an elevated temperature during step (b). Preferably, the process further comprises the addition of seed crystals of Compound of Formula la during or after step (b). Preferably, the temperature is about 30°C to about 45°C.

In another embodiment, the process further comprises granulating the compound of Formula **I** at an elevated temperature. Perferably, the temperature is about 30°C to about 45°C.

In a third aspect, the invention is directed to a process for preparing the compound of Formula **I**, 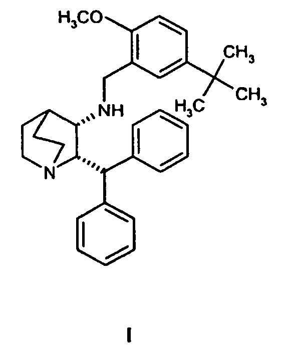 comprising removing the camphorsulfonate salt of a compound of **Ib,** 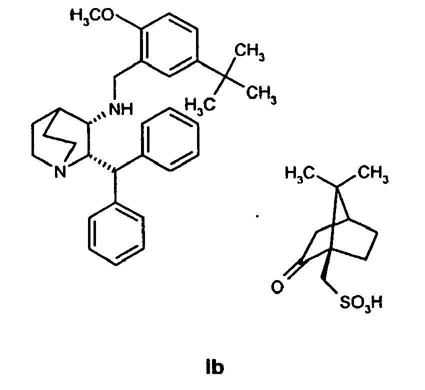 to provide the compound of Formula **I.**

In a preferred embodiment, the process further comprises reducing a compound of **IXa,** 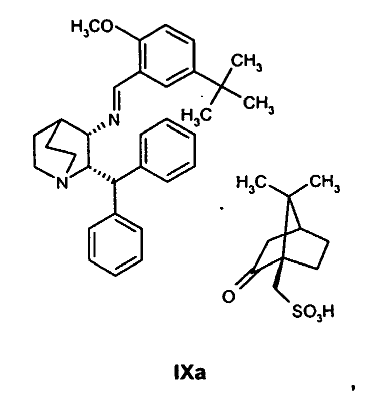 to provide the compound of Formula **Ib** so formed.

In a preferred embodiment, the process further comprises reacting a compound of Formula **VII,** 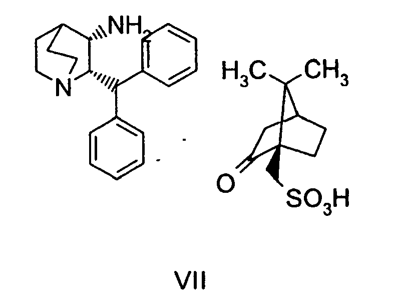 with a compound of Formula **VIII,** 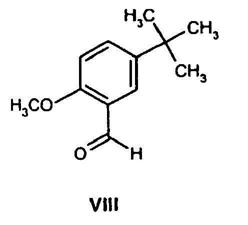 to provide the compound of formula **IXa** so formed.

In a preferred embodiment, the process further comprises deprotecting a compound of Formula **VIa,** 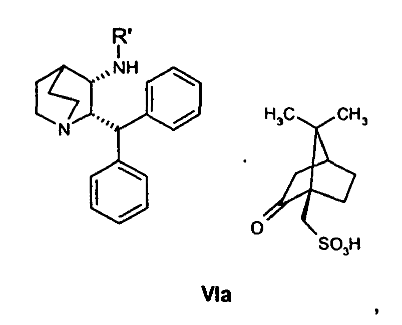 wherein R' is a protecting group selected from benzyl, 4-methoxybenzyl, 2,4-dimethoxybenzyl or triphenylmethyl, to provide the compound of Formula **VII** so formed.

In a preferred embodiment, the process further comprises treating the compound of Formula **I** with citric acid to form a compound of Formula **la**,

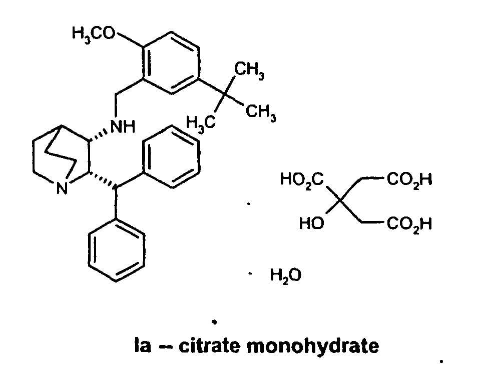

In a fourth aspect, the invention is directed to a compound of the Formula Vla,

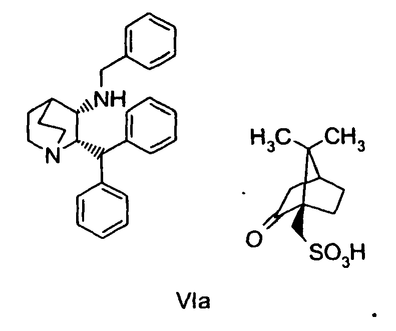

### BRIEF DESCRIPTION OF THE DRAWINGS

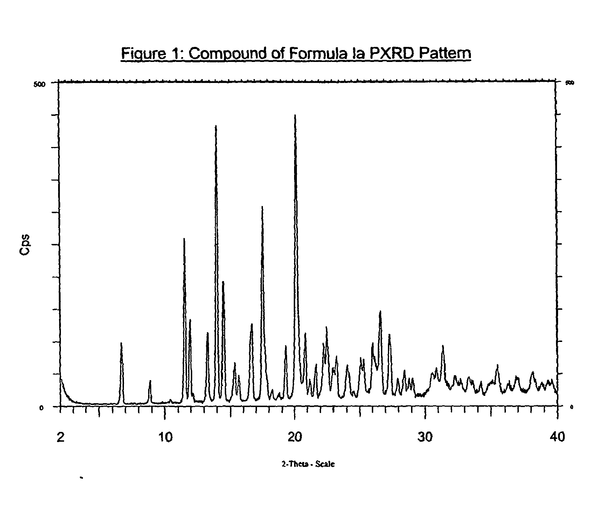

**Figure 1:** Compound of Formula la PXRD Pattern

### DESCRIPTION OF INVENTION

In general, the compound of Formula **I** may be prepared by methods that include processes known in the chemical arts, particularly in light of the description contained herein. Certain processes for the manufacture of the compound of Formula **I** of this invention are illustrated by the following reaction schemes. Other processes are described in the experimental section. Some of the starting compounds for the reactions described in the schemes and examples are prepared as illustrated in Preparation A and Preparation B. All other starting compounds may be obtained from general commercial sources, such as Sigma-Aldrich Corporation, St. Louis, MO, or may be prepared using methods known in the chemical literature.

The following reaction Schemes illustrate one possible preparation of the compound of the present invention. One skilled in the art would recognize that other protecting groups, other than benzyl, could also be utilized to prepare protecting group variations of the compound of Formula **VIa.** For example, other possible protecting groups are 4-methoxybenzyl, 2,4-dimethoxybenzyl, and triphenylmethyl.

Preparation A and Preparation B schemes depict alternative preparations of the starting compound, compound of Formula **VIa,** later used in Schemes **I** and **II**, wherein benzyl is utilized as the protecting group.

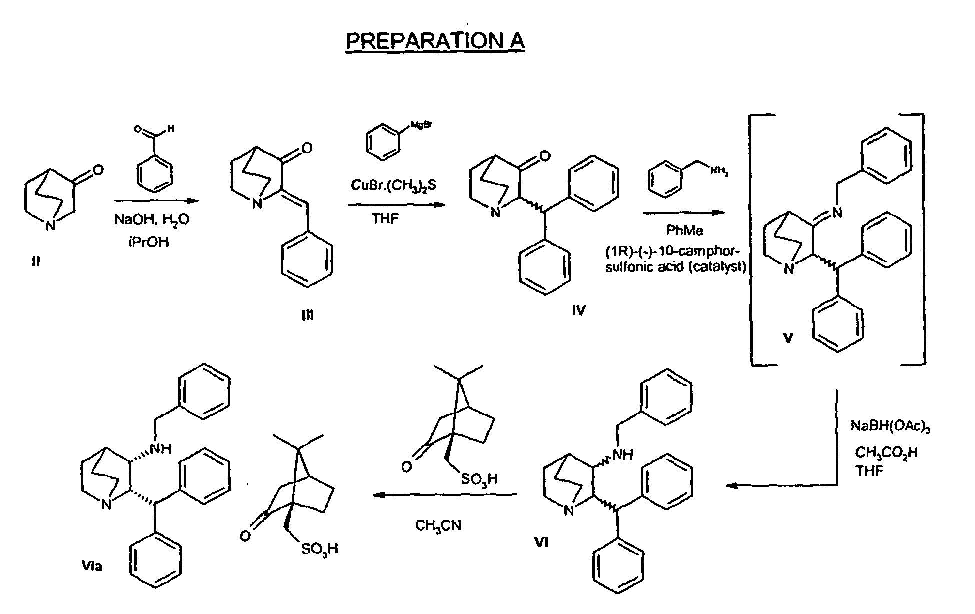

One possible synthesis of the compound of Formula **VIa** is detailed above in Preparation A. This route achieved optical purity of compound of Formula **VIa** by selective crystallization of the desired compound (cis 2S,3S form) as the (1*R*)-(-)10-camphorsulfonic acid salt from a racemic mixture of Compound **VI.** Up to 15% of the undesired *cis* enantiomer (cis 2R,3R form, typically 5-6%) and up to 2% of undesired trans diasteriomers (trans 2R, 3S and trans 2S, 3R forms, typically 1.3% observed), however, were produced in the process of synthesizing the compound of Formula **VIa** via this route.

With this particular approach to compound of Formula **VIa,** however, it is necessary to enhance and improve both optical and diastereomeric purity to achieve the desired quantity of compound of Formula I, prior to subjecting it to the synthetic route depicted in Scheme **I**.

An alternate synthesis to compound of Formula **V** and ultimately compound of Formula **Vla** is depicted in Preparation B. Preparation B is the subject of U.S. Non-provisional Application No. 10/679961, filed October 6. 2003. The text of the aforementioned application is hereby incorporated by reference in its entirety. As described above, one of ordinary skill in the art would recognize that different protecting groups, other than benzyl, could be utilized to prepare variations of the compound of Formula **Vla.** These variations are within the scope of the present invention.

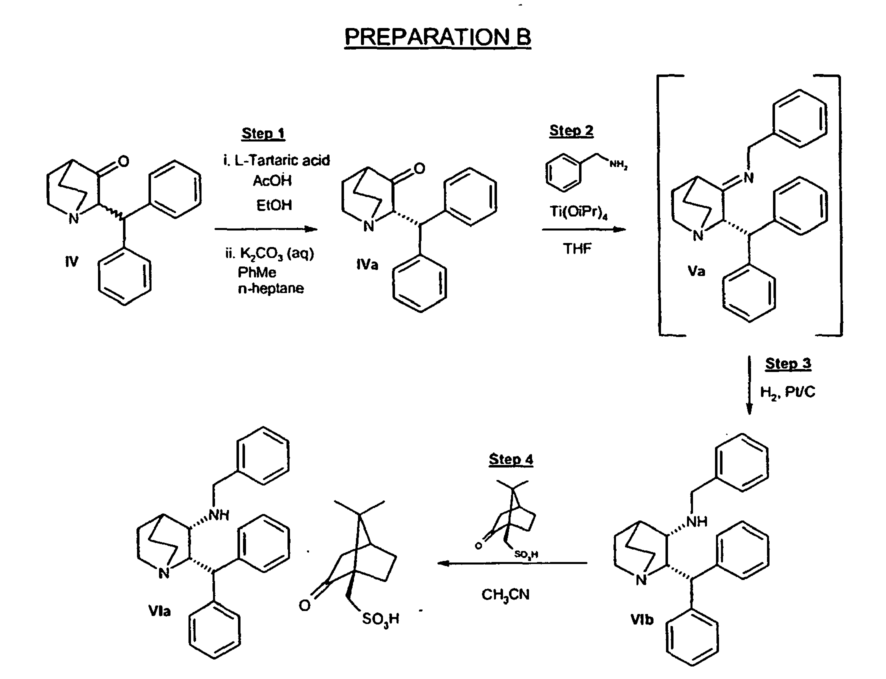

In Step 1 of Preparation B, the optical purification is first performed on the racemic ketone of Formula **IV,** the latter being dynamically resolved as the L-tartaric acid salt wherein the undesired (2*R*)-enantiomer is racemized under the reaction conditions to ultimately give the desired (2*S*)-enantiomer in greater than 50% yield. Optically pure compound of Formula **IVa** (up to 98% ee) was then reacted with benzyl amine under Schiff base-forming conditions to provide the imine intermediate, compound of Formula Va, which was catalytically reduced in a stereoselective manner to cis compound of Formula **Vlb.** The compound of Formula **Vla** is produced in a higher optical (enantiomeric) purity when compound **Vlb** it is converted to the (1*R*)-(-)-camphorsulfonate salt, eliminating the need for recrystallization of compound **VIa** to enhance stereochemical purity, when synthesized via the route described in Preparation B

The following reaction Scheme **I** illustrates an example of the preparation of the compound of Formula **Ia** from the compound of Formula **VIa**, as prepared via Preparation A.

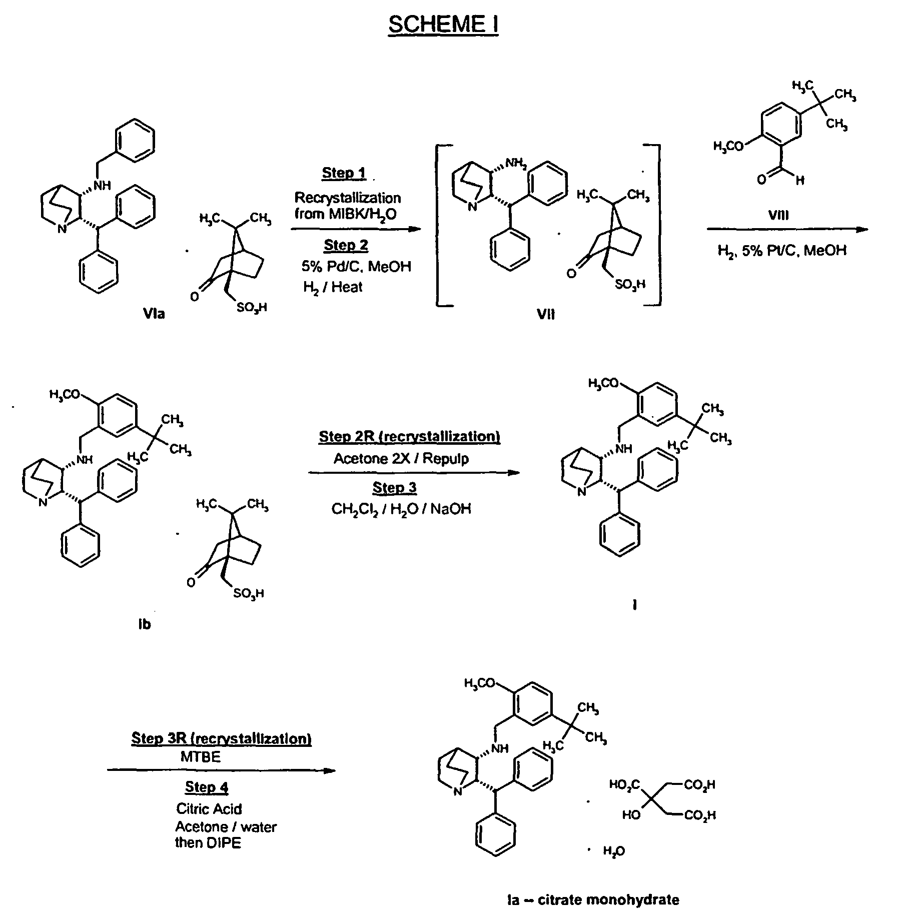

The compound of Formula **Vla** required additional purification to mimimize the presence of the undesired (cis 2R-3R)-enantiomer for use in the manufacture of compound of Formula **I**. Accordingly, in Step 1 of Scheme **I**, two successive recrystallizations of compound of Formula **VIa** were performed in 4-methyl-2-pentanone ("MIBK").

Compound **VIa** (50 g) was suspended in 10 volumes (500 mL) of 10% v/v water/MIBK solution) and heated to about 88-90°C for up to about 2 hours. The solution was cooled and the product isolated by filtration. The solvent wet product was re-suspended in 10 volumes of aqueous MIBK and heated again to about 88-90°C for up to two hours. The solution was then cooled to about 20-25°C and the product was isolated by filtration, washed with 0.5 volumes of MIBK and was then dried to yield Compound **Vla** in high enentiomeric purity (less than 0.2% of undesired enantiomer) in typically 83-85% yield.

In Step 2 of Scheme I, the compound of Formula **VIa** was catalytically deprotected, in this case, debenzylated, with a suitable catalyst, such as, palladium on carbon, palladium hydroxide on carbon, platinum on carbon, palladium on calcium carbonate , or palladium on alumina (Al₂O₃), in a solvent, such as methanol or isopropanol (propan-2-ol, "IPA") to provide compound of Formula **VII** in situ. In this particular synthesis, it was not necessary to isolate the intermediate compound **VII.** Instead, compound **VII** was reacted with compound **VIII** and hydrogen in the presence of a suitable catalyst, such as palladium on carbon, palladium hydroxide on carbon, platinum on carbon, palladium on calcium carbonate , or palladium on alumina (Al₂O₃), to provide compound **Ib.**

Compound **Ib** was recrystallized using acetone as solvent to provide purified compound **Ib.** Compound **I** was then prepared from compound **Ib** by basification using aqueous sodium hydroxide and extraction into dichloromethane followed by recrystallisation from tertiary-butyl methyl ether. Compound **I** was then suspended in a mixture of acetone and water, and citric acid was added followed by diisopropyl ether. The resultant solid was then collected by filtration, washed with diisopropyl ether and then dried to give compound **la.**

The following reaction Scheme **II** illustrates an alternative preparation of the compound of Formula **I** citrate monohydrate from the compound of Formula **Vla,** with improved yield from about 68% to about 76% Furthermore, the reaction of Scheme **II** is improved in that intermediary compounds (bracketed) do not require isolation, before proceeding forward to the next synthetic step.

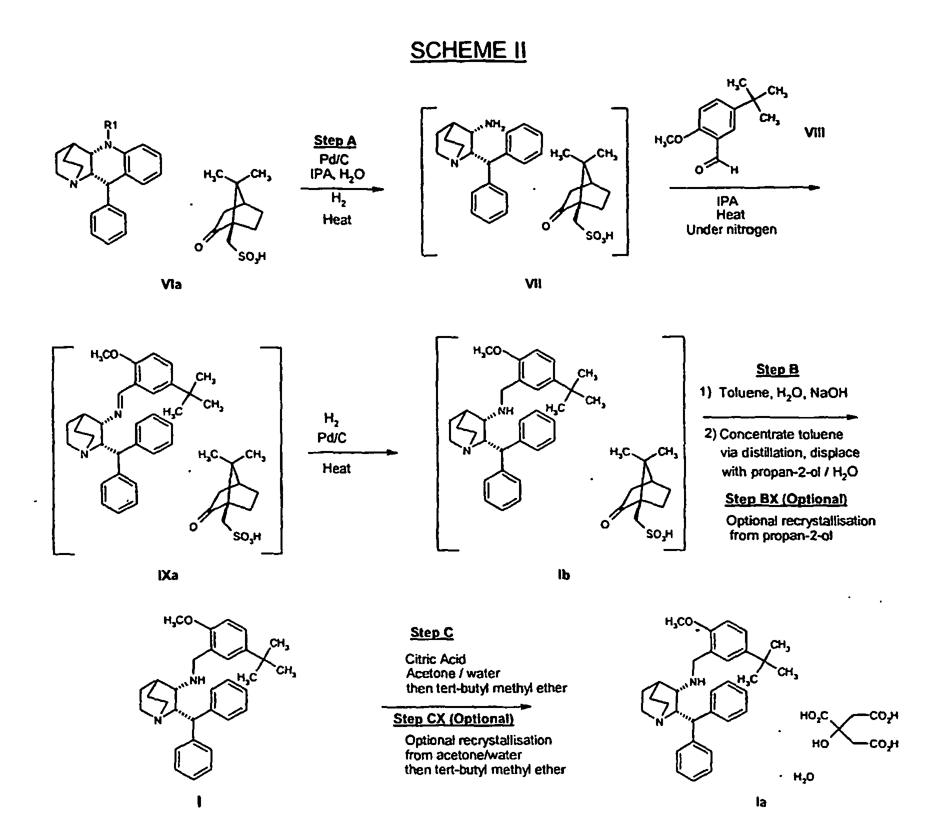

In Step A of Scheme **II**, a mixture of compound of Formula **VIa,** wherein R¹ is a protecting group such as benzyl, 4-methoxybenzyl, 2,4-dimethoxybenzyl, or triphenylmethyl, in an alcoholic solvent such as methanol, ethanol or n-propanol but preferably propan-2-ol, optionally also in the presence of water, was hydrogenated over a palladium on carbon catalyst at elevated temperature (typically 75-80° C) and pressure (typically 50 psig hydrogen). One skilled in the art would appreciate that other catalysts may be suitable, such as palladium on carbon, palladium hydroxide on carbon, platinum on carbon, palladium on calcium carbonate, or palladium on alumina (Al₂O₃).

Once formation of the intermediate, compound **VII,** was complete (about 1 hour) the compound of Formula **VIII,** typically as a solution in the respective alcoholic solvent, such as methanol, ethanol etc. (preferably in propan-2-ol (isopropanol, "IPA")) was added to the reaction, without isolating the compound of Formula **VII,** and the mixture stirred optionally at elevated temperature, from about 30°C to about 120°C, under an atmosphere of nitrogen. Once sufficient intermediate compound **IXa** was formed, the nitrogen atmosphere was replaced with hydrogen. The reaction was then stirred optionally at elevated temperature (about 30-120°C) and at elevated pressure (typically 50 psig) until the formation of the compound **Ib** was complete (typically 18 hours). The reaction mixture was then cooled (about 20-25°C) and the hydrogen gas vented. The palladium on carbon catalyst was removed by filtration, and the resultant solution of compound **Ib** was taken directly into Step **B**.

In Step **B** of the reaction scheme **II**, the solution of compound **Ib,** typically in a mixture of propan-2-ol and water, was concentrated by distillation followed by the addition of toluene. The mixture was then concentrated again by distillation, adding additional toluene and water as necessary during distillation until sufficient isopropanol had been removed from the mixture and an appropriate solution volume obtained (typically, 2-4 volumes per kg of compound **Ib).** Water and toluene were added as necessary (typically about 3.5 volumes of water and about 5 volumes of toluene). One skilled in the art would appreciate that other solvents, other than toluene, such as methylene chloride, ethyl acetate, isopropyl acetate or tert-butyl methyl ether, could be utilized. The pH was adjusted to an appropriate point (about 11.5 to 13.5) by the addition of aqueous sodium hydroxide and if necessary aqueous hydrochloric acid with stirring.

Once an appropriate pH has been obtained, the aqueous phase is removed by separation. The product-containing organic phase was then concentrated by distillation. A mixture of propan-2-ol and water was added and the mixture concentrated again by distillation. The addition of water and propan-2-ol and subsequent concentration by distillation was repeated as necessary until sufficient toluene (typically less than 3% w/w toluene by GC analysis) has been removed from the mixture and an appropriate solution volume has been obtained (about 4 volumes with respect to compound **Ib),** resulting in a composition of the solvent in the final granulation slurry of typically greater than 80% w/w propan-2-ol, less than 20% w/w water and less than 3%w/w toluene.

Once sufficient toluene has been removed, the mixture was cooled until crystallization occurs (typically 70-75°C). The resultant suspension was cooled further (typically to 20-25°C) and granulated for a period of time before being optionally cooled further to about 0-5°C and stirred for a period of time. The solid was collected by filtration, and the filter cake washed with propan-2-ol and dried under vacuum at elevated temperature (typically 45-55°C) to provide compound of formula **I,** as a crystalline solid. One skilled in the art would appreciate that other solvents, other than propan-2-ol, such as methanol, ethanol, n-propanol, acetonitrile, isopropyl acetate, tertiary-amyl alcohol and 4-methyl-2-pentanone could be utilized.

As outlined in the optional Step BX of the reaction scheme, which is not typically required, compound I may be further purified. Compound **I** was suspended in propan-2-ol and the mixture heated at reflux to give a solution. The mixture was then heated at an elevated temperature below the reflux temperature (about 70-75°C) for about 1 hour during which time crystallization typically occurs. The resultant suspension was maintained at this temperature for a period of about 1 to 2 hours and then cooled (to about 20-25°C). After stirring at ambient temperature for a period of time (typically 1-18 hours), the solid was collected by filtration. The filter cake was washed with propan-2-ol and then dried under vacuum at elevated temperature (about 45-55°C) to provide a purified compound **I,** as a crystalline solid. One skilled in the art would appreciate that other solvents, other than propan-2-ol, such as methanol, ethanol, n-propanol, acetonitrile, isopropyl acetate, tertiary-amyl alcohol and 4-methyl-2-pentanone could be utilized.

In Step C of the reaction scheme, compound **I** (1 molar equivalent) andanhydrous citric acid (typically 1.1 molar equivalents) were combined in mixture of acetone (typically about 8-10 volumes) and water (typically about 0.4 volumes), and the resultant solution filtered. More acetone (typically about 2 volumes) was then added to wash the transfer equipment through. To the filtrate was added a filtered ether solvent such as methyl tertiary-butyl ether (tert-butyl methyl ether, "MTBE") or isopropyl ether ("IPE") (typically about 10 volumes), optionally at elevated temperature (30-45°C). Once crystallization occured, which may optionally be initiated by the addition of some seed crystals, the mixture was granulated for a period of time (typically 18 hours), typically at 20-25°C but optionally at elevated temperature (30-45°C) for a portion of this time. The solid is then collected by filtration. The filter cake was washed with the respective filtered ether solvent and then dried at a temperature less than 60°C (room temperature, if using isopropyl ether) under vacuum optionally with no air or nitrogen bleed to provide compound **la,** the citrate monohydrate, as a crystalline solid. The product may then be optionally milled or sieved.

In optional Step CX, the purity of compound **Ia** may be improved by dissolving **Ia** in a mixture of acetone (typically 7 volumes) and water (typically 0.3 volumes) at elevated temperature (about 35-50°C). The mixture was then cooled (to about 20-35°C) and optionally filtered. To the resulting mixture was then added a filtered ether solvent, such as tert-butyl methyl ether or isopropyl ether, optionally at elevated temperature (about 30-40°C). Once crystallization occured, which may optionally be initiated by the additions of some seed crystals, the mixture was granulated for a period of time (typically 18 hours), typically at 20-25°C but optionally at elevated temperature (30-45°C) for a portion of this time. The solid was then collected by filtration. The filter cake was washed with the respective filtered ether solvent and then dried at a temperature less than 60°C (room temperature, if using isopropyl ether) under vacuum optionally with no air or nitrogen bleed to provide compound la, the citrate monohydrate, as a crystalline solid. The product may then be optionally milled or sieved.

Other pharmaceutically acceptable salts, other than the citrate, may be utilized. For example, malate, maleate, mesylate, lactate, and hydrochloride salts or their *in situ* equivalents may be prepared by adding equimolar amount of the appropriate acid to the compound **I**, free base solutions.

### GENERAL EXPERIMENTAL PROCEDURES

### Recrystallization of compound of Formula VIa (Scheme I).

To a 3-L round-bottomed flask, equipped with a mechanical stirrer, reflux condenser, thermometer and thermostated oil bath, was added compound **VIa** (200 grams), methyl-isobutylkentone ("MIBK") (1900 mL) and 100 mL of water. The suspension was gradually heated to reflux under stirring, about 30 minutes, and kept at 88-90°C for about 15-30 minutes, until achieving a complete solution. (The MIBK/water azeotrope boils at about 88 °C.) At this stage, the mixture was biphasic with a small amount of water undissolved.

The mixture was slowly cooled, while stirring, to room temperature (about 20-25 °C) in about 2 hours. The product began to precipitate at about 60 °C. The suspension was granulated at 20-25°C for about 2-3 hours, but also could be held overnight at 20°C, and the precipitate was filtered and washed with about 100 mL of MIBK.

The wet cake (about 220-230 grams) was recrystallized, as described above, using 1700 mL of MIBK and 91 mL of water. The suspension was again granulated for at least 3 hours or overnight at 20-25 °C. The product is filtered and washed with MIBK (100 mL). The purified compound **VIa** was dried in an air tray-drier at 50°C, until constant weight was obtained (about 18 hours), providing a white crystalline purified solid, compound **VIa.** Yield 85%. Chiral purity: (2R-cis) enantiomer 0.3-0.5%.

### Preparation of (2S,3S)-2-Benzhydryl-N-(5-tert-butyl-2-methoxybenzyl) quinuclidin-3-amine (1R)-10-camphorsulfonate, compound of formula Ib, as a solution in propan-2-ol/water, (Step A, Scheme II).

To a mixture of (2*S*,3*S*)-2-benzhydryl-*N*-benzylquinuclidin-3-amine (1*R*)*-*10-camphorsulfonate (compound of formula Vla, 18.0 kg, 29.3 moles) and water (18.0 kg) in propan-2-ol (57.9 kg) was added 5% palladium on carbon, 50% water wet (2.88kg) and the resultant mixture was hydrogenated at 50psi hydrogen pressure at 75-80°C for 4 hours. The mixture was then cooled to 15-20°C, and the hydrogen atmosphere was replaced by nitrogen (5psi). To this mixture was then added a solution of 5-*tert*-butyl-2-methoxybenzaldehyde (6.47 kg, 33.7 moles) in propan-2-ol (6.47 kg). The addition line was then washed with propan-2-ol (4.24kg) and this was added to the reaction mixture, which was then stirred at 75-80°C for 2 hours under a nitrogen atmosphere. The resultant mixture was then cooled to 30-40°C, and the nitrogen atmosphere was replaced by hydrogen (50psi). The mixture was then hydrogenated at 50psi hydrogen pressure at 75-80°C for 3.5 hours, after which time the reaction was cooled to 25-30°C and the hydrogen pressure was reduced to 10psi for 10 hours for convenience. The reaction was then re-pressurised with hydrogen (50psi) and heated to 75-80°C for 11.5 hours, after which time the reaction was again cooled to 25-30°C and the hydrogen pressure was reduced to 10psi for 10 hours for convenience. The reaction was then re-pressurised with hydrogen (50psi) and heated to 75-80°C for 3 hours so that the total reaction time at 75-80°C was 18 hours. The reaction was then cooled to 15-20°C and the hydrogen atmosphere was replaced by nitrogen. The resultant suspension was then filtered to remove the catalyst, and the filter cake was washed with propan-2-ol (19.8 kg). The combined filtrate and washings, comprising a solution of (2*S*,3*S*)-2-benzhydryl-*N*-(5-*tert*-butyl-2-methoxybenzyl)quinuclidin-3-amine (1*R*)-10-camphorsulfonate (compound of formula lb) in propan-2-ol/water, were taken as such into the next step, denoted as Step B.

### Preparation of (2S,3S)-2-Benzhydryl-N-(5-tert-butyl-2-methoxybenzyl) quinuclidin-3-amine, compound of formula I (Step B, Scheme II).

Three solutions of (2*S*,3*S*)-2-benzhydryl-*N*-(5-*tert*-butyl-2-methoxybenzyl)quinuclidin-3-amine (1*R*)-10-camphorsulfonate (compound of formula lb) in propan-2-ol/water, each prepared from (2S,3S)-2-benzhydryl-N-benzylquinuclidin-3-amine (1*R*)-10-camphorsulfonate (compound of formula **Vla,** 18kg, 29.3 moles) and 5-*tert*-butyl-2-methoxybenzaldehyde (6.47 kg, 33.7 moles) using the Step A (Scheme II) process as previously described, were combined to give a total approximate volume of 430 L of solution. This was then concentrated to a volume of approximately 160 L by distillation under vacuum. Toluene (266kg) was then added and the resultant mixture was concentrated by atmospheric pressure distillation until the volume was approximately 160 L. Water (216 kg) and toluene (250 kg) were then added, and the mixture was then cooled to 20-25°C. The pH of the aqueous phase was adjusted to pH 12.5-12.9 by the addition of aqueous sodium hydroxide with agitation. The aqueous phase was then removed, and the organic phase was concentrated to a volume of approximately 160 L by distillation under vacuum. Water (30.8 kg) and propan-2-ol (218 kg) were then added and the resultant mixture was then concentrated to a volume of approximately 160 L by distillation at atmospheric pressure. At this point, the mixture was held at 25-35°C for 18 hours for convenience. Water (33.8 kg) and propan-2-ol (218 kg) were then added, and the mixture was concentrated to a volume of approximately 160 L by distillation at atmospheric pressure. Water (21 kg) and propan-2-ol (141 kg) were then added, and the mixture was concentrated to a volume of approximately 160 L by distillation at atmospheric pressure. Whilst maintaining the temperature of the reaction mixture above 75°C, propan-2-ol (97 kg) was then slowly added, and the resultant mixture was then cooled to 70°C for 1.5 hours during which time crystallization occurred. The resultant suspension was then cooled to 20-25°C over 5 hours and was stirred at this temperature for 11 hours. The solid was then collected by filtration, and the filter cake was washed twice with propan-2-ol (17 kg and 34 kg). The resultant solid was then dried under vacuum at 50°C to give the title compound (35.3 kg) as a colourless solid. ¹H-NMR (300 MHz, CDCl₃, 30°C) δ : 7.33 (2H, br d), 7.27-7.10 (8H, m), 7.10-7.01 (1H, m), 6.92 (1H, d), 6.65 (1H, d), 4.51 (1H, d), 3.73-3.52 (5H, m), 3.26-3.09 (2H, m), 2.77 (1H, dd), 2.82-2.73 (2H, m), 2.59 (1H, br t), 2.15-2.06 (1H, m), 2.01-1.87 (1H, m), 1.73-1.60 (1H, m), 1.60-1.43 (1H, m), 1.32-1.19 (10H, m). LRMS (positive atmospheric pressure chemical ionization): m/z [MH⁺] 469.

### Optional purification of (2S,3S)-2-benzhvdryl-N-(5-tert-butyl-2-methoxybenzyl) quinuclidin-3-amine, compound of formula I (Step BX, Scheme II).

A suspension of (2S,3S)-2-benzhydryl-N-(5-tert-butyl-2-methoxybenzyl)quinuclidin-3-amine (compound of formula I, 70 g) in propan-2-ol (350 mL) was heated to reflux for 1 hour to give a solution. The resultant mixture was then cooled to 70-75°C for 2 hours during which time crystallization occurred, and the resultant suspension was then cooled to 20-25 °C over approximately 4 hours. The mixture was then cooled to 0-3°C for 0.5 hours and the solid was then collected by filtration. The filter cake was then washed twice with propan-2-ol (70 mL each) and the resultant solid was dried under vacuum at 50°C to give the title compound (67.7 g) as a colourless solid.

### Preparation of (2S,3S)-2-benzhydryl-N-(5-tert-butyl-2-methoxybenzyl) quinuclidin-3-amine citrate monohydrate, compound of formula la (Step C, Scheme II).

A solution of (2S,3S)-2-benzhydryl-*N*-(5-*tert*-butyl-2-methoxybenzyl)quinuclidin-3-amine (33.95 kg, 72.4 moles) and anhydrous citric acid (15.3 kg, 79.7 moles) in a mixture of acetone (215 kg) and water (13.6 kg) was heated to 38-42°C. The resultant mixture was then transferred to another reactor via an in-line filter. The transfer line and filter were washed through with acetone (54 kg) and these filtered washings were added to the solution. The resultant mixture was then cooled to 20-25°C and filtered *tert*-butyl methyl ether (252kg) was added portionwise over a period of approximately 35 minutes. The resultant suspension was then granulated at 20-25°C for approximately 20 hours. The solid was then collected by filtration on an agitated filter-dryer and the filter cake was washed twice with filtered *tert*-butyl methyl ether (50kg each). The resultant solid was then dried at 35°C under vacuum with agitation to give the title compound (44.4kg) as a colourless solid. The product was then milled. ¹H-NMR (500 MHz, d⁴-methanol, 30°C) δ : 7.46 (2H, d), 7.45 (2H, d), 7.37 (4H, m), 7.31 (1H, m), 7.29 (1H, m), 7.24 (1H, dd), 6.95 (1 H, d), 6.76 (1H, d), 4.75 (1H, dd), 4.71 (1H, d), 3.76 (1H, m), 3.57 (1H, d), 3.55 (3H, s), 3.37 (1H, m), 3.31 (1H, m), 3.26 (1H, m), 3.24 (1H, d), 3.10 (1H, t), 2.83 (2H, d), 2.75 (2H, d), 2.51 (1H, m), 2.35 (1H, m), 2.11 (1H, m), 2.06 (1H, m), 1.85 (1H, m), 1.29 (9H, s). ¹³C NMR (125.7 MHz, d⁴-methanol, 30°C) δ : 179.4, 175.0, 156.8, 144.0, 141.5, 141.4, 131.1, 130.6, 129.4, 128.9, 128.7, 128.3, 128.2, 127.2, 126.4, 111.0, 74.0, 64.7, 56.1, 54.2, 50.4, 48.5, 48.3, 44.9, 43.8, 34.8, 32.9, 25.3, 22.2, 18.1. LRMS (ES+): m/z [MH⁺] 469.

The solid Compound of Formula **Ia** prepared by this process exhibited the following Powder X-Ray Diffaction characteristics:

**Table I: Compound of Formula la Peak Position Table**

| °2-Theta | Intensity % | °2-Theta | Intensity % | °2-Theta | Intensity % |
|---|---|---|---|---|---|
| 6.66 | 36.80 | 20.57 | 20.00 | 26.34 | 18.00 |
| 8.81 | 8.40 | 20.86 | 37.10 | 26.62 | 25.30 |
| 11.51 | 41.90 | 21.18 | 12.30 | 27.36 | 16.20 |
| 11.90 | 55.40 | 21.64 | 20.20 | 27.97 | 5.80 |
| 13.23 | 21.70 | 22.30 | 12.00 | 28.47 | 8.20 |
| 13.99 | 100.00 | 22.51 | 28.90 | 28.81 | 6.50 |
| 14.47 | 26.40 | 22.96 | 18.80 | 30.57 | 7.70 |
| 15.29 | 21.40 | 23.21 | 22.50 | 31.00 | 9.40 |
| 15.61 | 17.60 | 24.01 | 5.90 | 31.42 | 21.70 |
| 16.65 | 42.40 | 24.18 | 8.60 | 31.79 | 6.50 |
| 17.54 | 53.90 | 25.09 | 15.80 | 32.33 | 5.90 |
| 17.81 | 19.80 | 25.32 | 9.50 | 32.74 | 6.70 |
| 18.22 | 7.00 | 25.69 | 5.30 | 33.32 | 5.50 |
| 19.30 | 15.50 | 26.03 | 11.40 | 35.19 | 6.40 |
| 20.18 | 79.90 | 26.19 | 14.30 | | |

### Powder X-Rav Diffraction pattern acquisition details.

The powder X-ray diffraction pattern was determined using a SIEMENS D5000 powder X-ray diffractometer fitted with an automatic sample changer, a theta-theta goniometer, automatic beam divergence slits, a secondary monochromator and a scintillation counter. The sample was prepared for analysis by packing the powder into 12mm diameter, 0.25mm deep cavity that had been cut into silicon wafer specimen mount. The specimen was rotated whilst being irradiated with copper K-alpha, X-rays (wavelength = 1.5406 Angstroms) with the X-ray tube operated at 40kV/40mA. The analyses were performed with the goniometer running in step-scan mode set for a 5 second count per 0.02° step over a two theta range of 2° to 40°.

### Optional purification of (2S,3S)-2-benzhydryl-N-(5-tert-butyl-2-methoxybenzyl) quinuclidin-3-amine citrate monohydrate, compound of formula la (Step CX (Scheme II).

A mixture of (2S,3S)-2-benzhydryl-N-(5-tert-butyl-2-methoxybenzyl)quinuclidin-3-amine citrate monohydrate (38.47kg, 56.7 moles) and filtered water (11.5 kg) in filtered acetone (213kg) was heated to 38-42°C to achieve a solution, which was then cooled to 33-37°C. To this solution was then added filtered *tert*-butyl methyl ether (201 kg) over a period of approximately 35 minutes whilst maintaining the temperature at 33-37°C. The resultant suspension was then cooled to 20-25°C and was then granulated at this temperature for approximately 19 hours. The solid was then collected by filtration on an agitated filter-dryer and the filter cake was washed twice with filtered *tert*-butyl methyl ether (58 kg each). The resultant solid was then dried at 35°C under vacuum with agitation to give the title compound (32.9 kg) as a colourless solid. The product was then milled.

### Preferred Embodiments

1. A process for preparing the compound of Formula **Ib,** 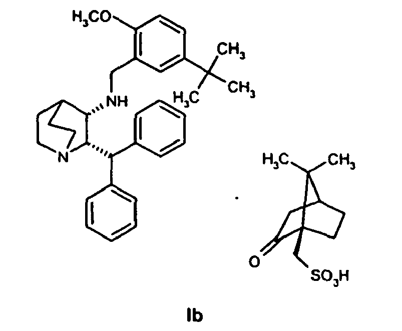 comprising:
   (a) deprotecting a compound of Formula **VIa,** 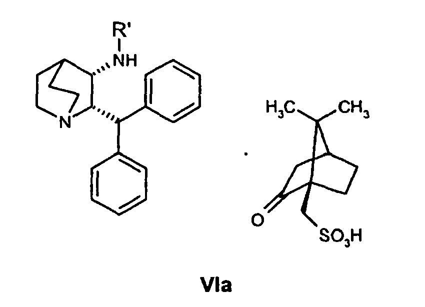 wherein R' is a protecting group, to provide a compound of Formula **VII;** 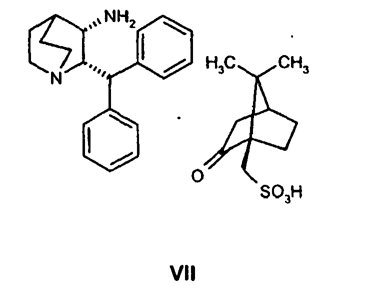
   (b) reacting the compound of formula **VII** so formed with a compound of formula **VIII,** 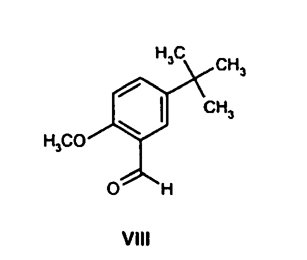 and performing a reductive amination to provide a compound of Formula **Ib,** 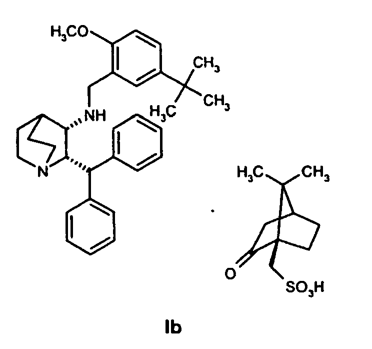
2. The process according to Preferred embodiment 1 further comprising removing the camphorsulfonate salt of the compound of Formula **Ib** to provide a compound of Formula **I**. 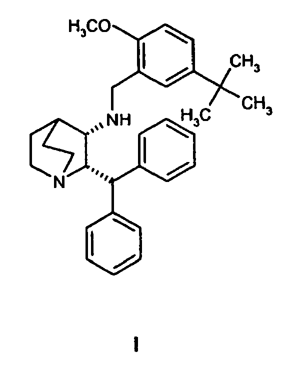
3. The process according to Preferred embodiment 2, wherein the protecting group is benzyl, 4-methoxybenzyl, 2,4-dimethoxybenzyl, or triphenylmethyl.
4. The process according to Preferred embodiment 3, wherein the deprotection is performed by catalytic hydrogenolysis with hydrogen.
5. The process according to Preferred embodiment 4, wherein the catalyst is palladium on carbon, platinum on carbon, palladium on calcium carbonate, or palladium on alumina (Al₂O₃).
6. The process according to Preferred embodiment 5, wherein the reductive animation is performed by formation of an imine followed by catalytic hydrogenation.
7. The process according to Preferred embodiment 6, wherein the hydrogenation catalyst is palladium on carbon, platinum on carbon, palladium on calcium carbonate, or palladium on alumina (Al₂O₃).
8. The process according to Preferred embodiment 7 further comprising treating the compound of Formula **I** with citric acid, forming the compound of Formula **Ia.** 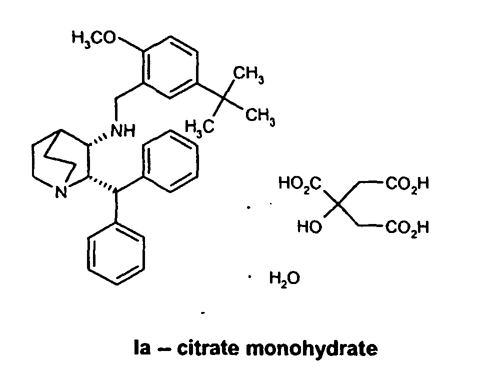
9. A process for preparing the compound of Formula **I**, 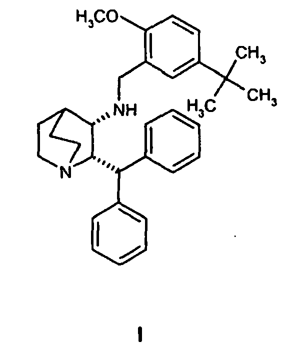 comprising:
   (a) debenzylating a compound of Formula **Vla** 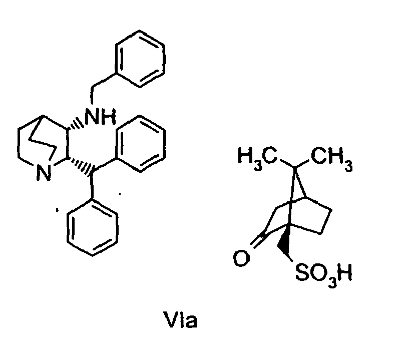 to provide a compound of Formula **VII;** 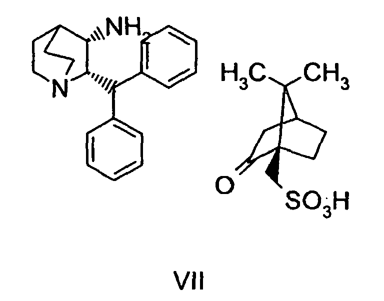
   (b) reacting the compound of formula **VII** so formed with a compound of formula **VIII,** 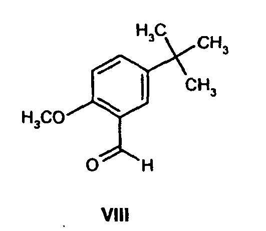 and performing a reductive amination to provide a compound of Formula **Ib,** 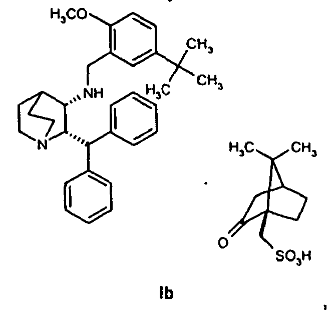 and
   (c) removing the camphorsulfonate salt of the compound of **Ib** to provide the compound of Formula **I.**
10. The process according to Preferred embodiment 9 wherein the debenzylation is performed by catalytic hydrogenation.
11. The process according to Preferred embodiment 10 wherein the catalyst is palladium on carbon, platinum on carbon, palladium on calcium carbonate, or palladium on alumina (Al₂O₃).
12. The process according to Preferred embodiments 9, 10 or 11 further comprising a reductive amination of step (b) that is performed by catalytic hydrogenation.
13. The process according to Preferred embodiment 12, wherein the catalyst is palladium on carbon, platinum on carbon, palladium on calcium carbonate, or palladium on alumina (Al₂O₃).
14. The process according to Preferred embodiment 13 further comprising isolating the compound of Formula **I.**
15. The process according to Preferred embodiment 14 wherein the isolation of the compound of Formula **I** occurs by acid counter ion exchange or basification followed by selective crystallization.
16. The process according to Preferred embodiment 15 wherein the crystallization is accomplished in a solvent selected from water, alcohols, ethers, hydrocarbons or mixtures thereof.
17. The process according to Preferred embodiment 16 wherein the solvent is isopropanol, toluene or water or mixtures thereof.
18. The process according to Preferred embodiment 15 wherein the basification is performed by the addition of an inorganic or organic reagent.
19. The process according to Preferred embodiment 18 wherein the reagent is sodium hydroxide, sodium carbonate or sodium bicarbonate.
20. The process according to Preferred embodiment 9 further comprising treating the compound of Formula **I** with citric acid, forming the compound of Formula la 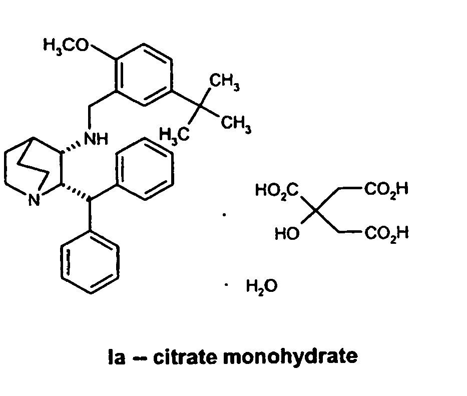
21. The process according to Preferred embodiment 20 further comprising the addition of acetone and water.
22. The process according to Preferred embodiment 21 further comprising
   (a) filtering the solution; and
   (b) adding a filtered ether solvent, providing a compound of Formula **Ia.**
23. The process according to Preferred embodiment 22 further comprising the additional step (c) of granulating the compound of Formula **Ia**.
24. The process according to Preferred embodiment 22 wherein the ether solvent is tert-butyl methyl ether.
25. The process according to Preferred embodiment 22 further comprising applying heat at an elevated temperature during step (b).
26. The process according to Preferred embodiment 22 further comprising the addition of seed crystals of Compound of Formula **Ia** during or after step (b).
27. The process according to Preferred embodiment 25 wherein the temperature is about 30°C to about 45°C.
28. The process according to Preferred embodiment 23 further comprising granulating the compound of Formula **I** at an elevated temperature.
29. The process according to Preferred embodiment 28 wherein the temperature is about 30°C to about 45°C.
30. A process for preparing the compound of Formula **I,** 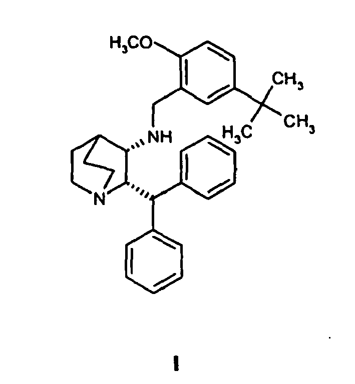 comprising removing the camphorsulfonate salt of a compound of **Ib,** 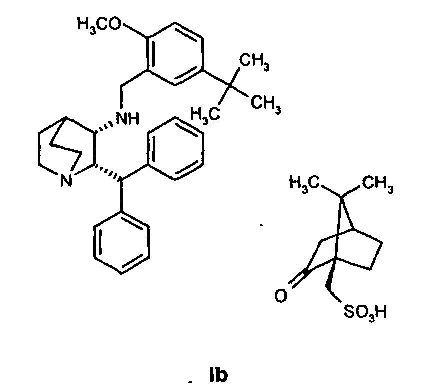 to provide the compound of Formula **I.**
31. The process according to Preferred embodiment 30 further comprising reducing a compound of **IXa,** 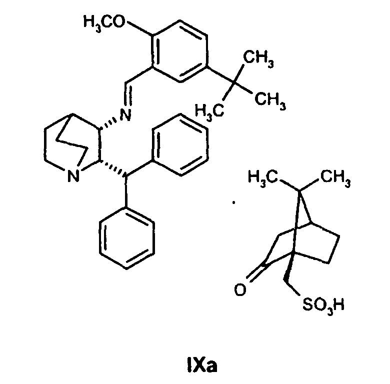 to provide the compound of Formula **Ib** so formed.
32. The process according to Preferred embodiment 31 further comprising reacting a compound of Formula **VII,** 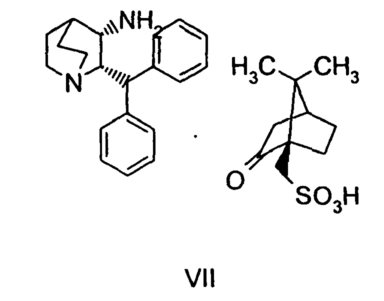 with a compound of Formula **VIII,** 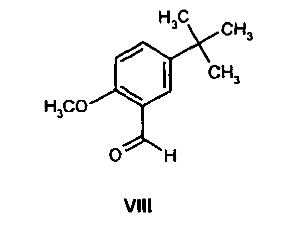 to provide the compound of formula **IXa** so formed.
33. The process according to Preferred embodiment 32 further comprising deprotecting a compound of Formula **VIa,** 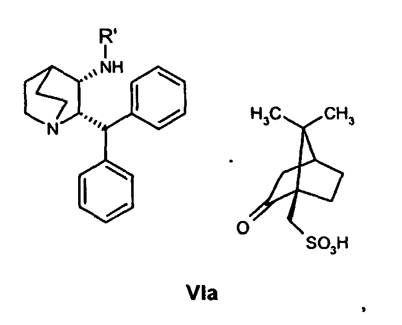 wherein R' is a protecting group selected from benzyl, 4-methoxybenzyl, 2,4-dimethoxybenzyl or triphenylmethyl, to provide the compound of Formula **VII** so formed.
34. The process according to Preferred embodiments 30, 31, 32 and 33 further comprising treating the compound of Formula **I** with citric acid to form a compound of Formula **la,** 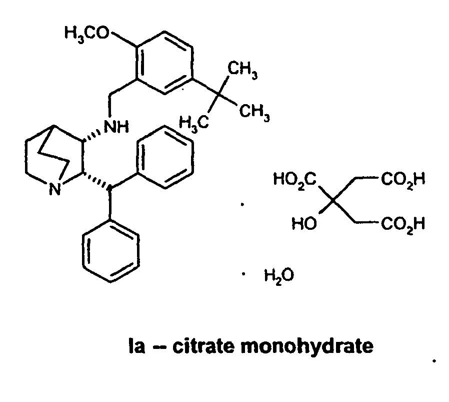
35. A compound of the Formula V**I**a

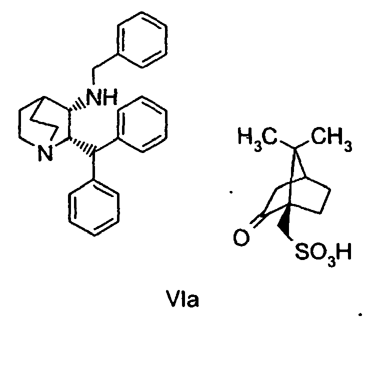

## Claims

1. A process for preparing the compound of Formula **Ib,** 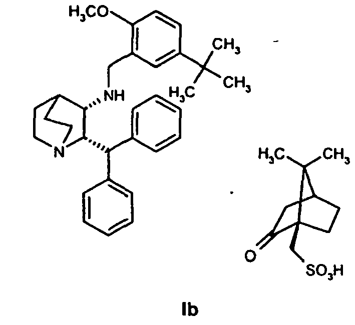 comprising:
(c) deprotecting a compound of Formula **VIa,** 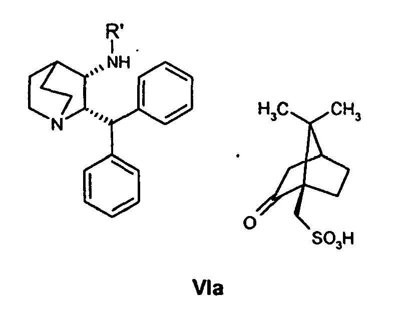 wherein R' is a protecting group, to provide a compound of Formula **VII;** 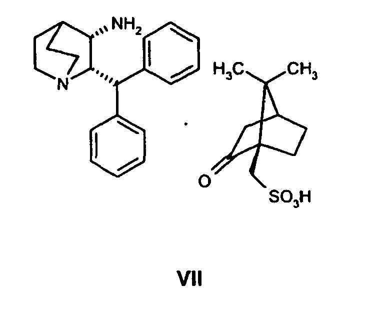
(d) reacting the compound of formula **VII** so formed with a compound of formula **VIII,** 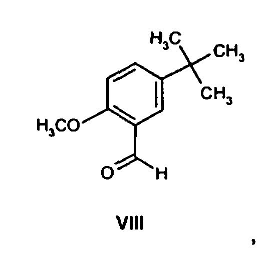 and performing a reductive amination to provide a compound of Formula **Ib,**

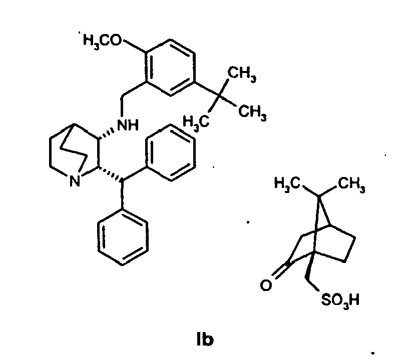

2. The process according to Claim 1 further comprising removing the camphorsulfonate salt of the compound of Formula **Ib** to provide a compound of Formula **I,**

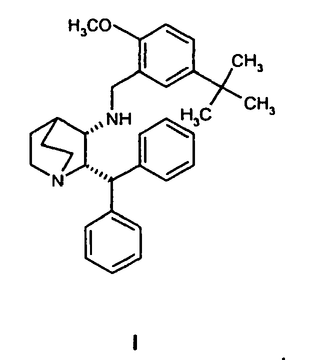

3. The process according to Claim 1 or Claim 2, wherein the protecting group is benzyl, 4-methoxybenzyl, 2,4-dimethoxybenzyl, or triphenylmethyl.

4. The process according to Claim 3, wherein the deprotection is performed by catalytic hydrogenolysis with hydrogen.

5. The process according to Claim 4, wherein the catalyst is palladium on carbon, platinum on carbon, palladium on calcium carbonate, or palladium on alumina (Al₂O₃).

6. The process according to any preceding Claim wherein the reductive animation is performed by formation of an imine followed by catalytic hydrogenation.

7. The process according to Claim 6, wherein the hydrogenation catalyst is palladium on carbon, platinum on carbon, palladium on calcium carbonate, or palladium on alumina (Al₂O₃).

8. The process according to any of Claims 2 to 7 further comprising isolating the compound of Formula **I.**

9. The process according to any of Claims 2 to 8 further comprising treating the compound of Formula **I** with citric acid, forming the compound of Formula **la**

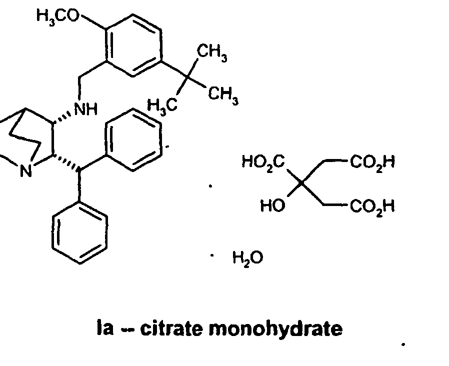

10. A compound of the Formula **VIa**,

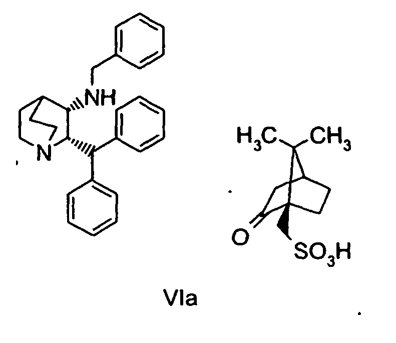

## Patentansprüche

1. Verfahren zur Herstellung der Verbindung der Formel Ib 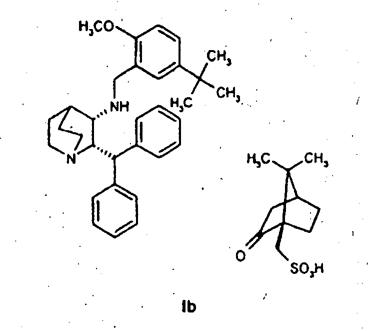 umfassend:
(c) das Entschützen einer Verbindung der Formel **VIa** 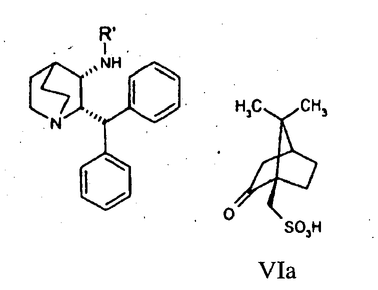 in der R' eine Schutzgruppe ist, um eine Verbindung der Formel **VII** zur Verfügung zu stellen 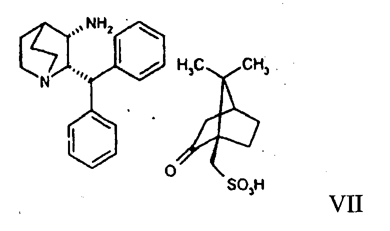
(d) Umsetzen der auf diese Weise hergestellten Verbindung der Formel **VII** mit einer Verbindung der Formel 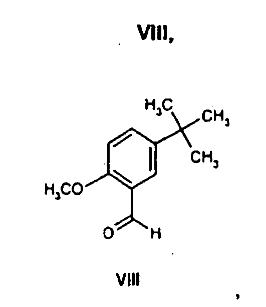 und Durchführen einer reduktiven Aminierung, um eine Verbindung der Formel Ib zur Verfügung zu stellen

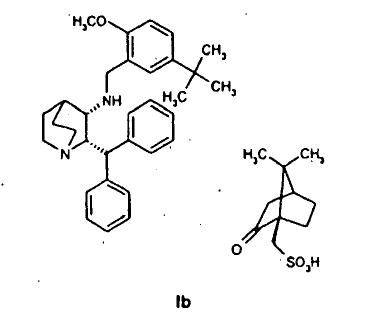

2. Verfahren nach Anspruch 1, das außerdem die Entfernung des Camphersulfonatsalzes der Verbindung der Formel Ib umfasst, um eine Verbindung der Formel **I** zur Verfügung zu stellen:

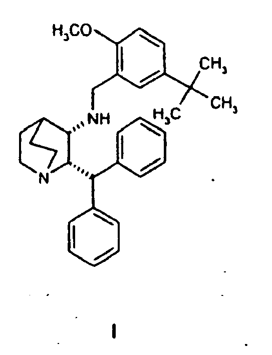

3. Verfahren nach Anspruch 1 oder 2, bei dem die Schutzgruppe Benzyl, 4-Methoxybenzyl, 2,4-Dimethoxybenzyl oder Triphenylmethyl ist.

4. Verfahren nach Anspruch 3, bei dem die Entschützung durch katalytische Hydrogenolyse mit Wasserstoff erfolgt.

5. Verfahren nach Anspruch 4, bei dem der Katalysator Palladium auf Kohlenstoff, Platin auf Kohlenstoff, Palladium auf Calciumcarbonat oder Palladium auf Aluminiumoxid (Al₂O₃) ist.

6. Verfahren nach einem der vorstehenden Ansprüche, bei dem die reduktive Aminierung durch Bildung eines Imins, gefolgt von einer katalytischen Hydrierung erfolgt.

7. Verfahren nach Anspruch 6, bei dem der Hydrierungskatalysator Palladium auf Kohlenstoff, Platin auf Kohlenstoff, Palladium auf Calciumcarbonat oder Palladium auf Aluminiumoxid (Al₂O₃) ist.

8. Verfahren nach einem der Ansprüche 2 bis 7, das außerdem die Isolierung der Verbindung der Formel I umfasst.

9. Verfahren nach einem der Ansprüche 2 bis 8, das außerdem die Behandlung der Verbindung der Formel I mit Citronensäure umfasst, um die Verbindung der Formel Ia 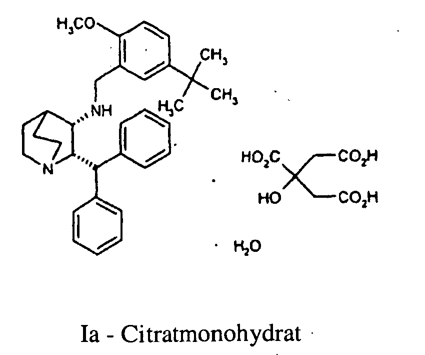 herzustellen.

10. Verbindung der Formel VIa

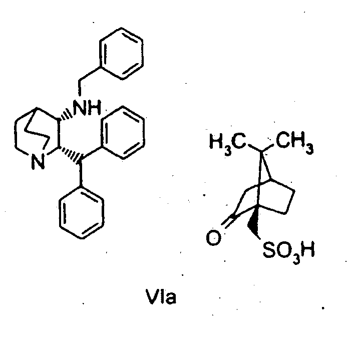

## Revendications

1. Procédé de préparation du composé de Formule **Ib** 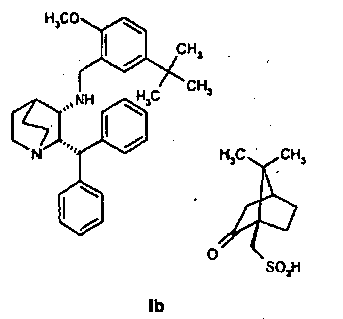 comprenant :
(c) la déprotection d'un composé de Formule **VIa** 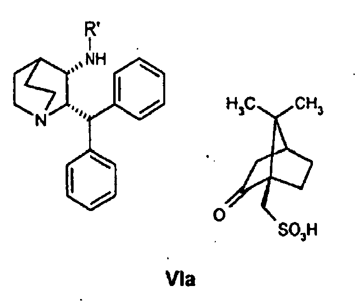 où R' est un groupe protecteur, pour donner un composé de Formule **VII** 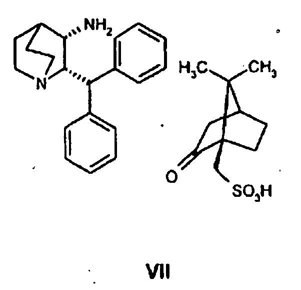
(d) la réaction du composé de Formule **VII** ainsi formé avec un composé de Formule **VIII** 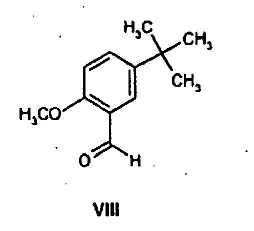 et la mise en oeuvre d'une amination réductrice pour donner un composé de Formule **Ib**

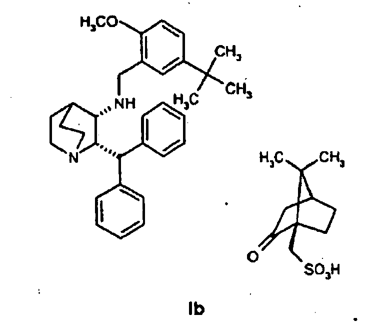

2. Procédé selon la revendication 1, comprenant en outre l'élimination du sel camphosulfonique du composé de Formule **Ib** pour donner un composé de Formule **I**

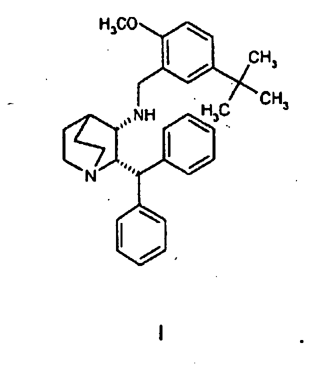

3. Procédé selon la revendication 1 ou la revendication 2, dans lequel le groupe protecteur est un groupe benzyle, 4-méthoxybenzyle, 2,4-diméthoxybenzyle ou triphénylméthyle.

4. Procédé selon la revendication 3, dans lequel la déprotection est mise en oeuvre par hydrogénolyse catalytique avec de l'hydrogène.

5. Procédé selon la revendication 4, dans lequel le catalyseur est du palladium sur carbone, du platine sur carbone, du palladium sur carbonate de calcium ou du palladium sur alumine (Al₂O₃).

6. Procédé selon l'une quelconque des revendications précédentes, dans lequel l'amination réductrice est mise en oeuvre par formation d'une imine suivie d'une hydrogénation catalytique.

7. Procédé selon la revendication 6, dans lequel le catalyseur d'hydrogénation est du palladium sur carbone, du platine sur carbone, du palladium sur carbonate de calcium ou du palladium sur alumine (Al₂O₃).

8. Procédé selon l'une quelconque des revendications 2 à 7, comprenant, en outre, l'isolement du composé de Formule **I.**

9. Procédé selon l'une quelconque des revendications 2 à 8, comprenant, en outre, le traitement du composé de Formule **I** avec de l'acide citrique, formant ainsi le composé de Formule Ia

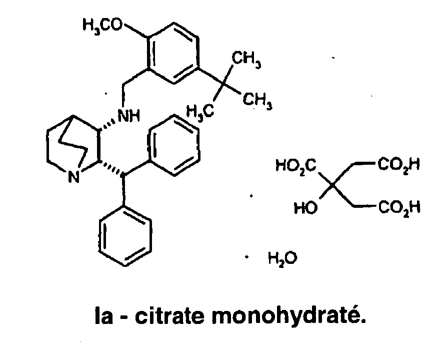

10. Composé de Formule **VIa**
